# EUROPEAN PATENT APPLICATION

(11) **EP 1 034 734 A1**
(43) Date of publication of application: **13.09.2000**
(21) Application number: 00650017.7
(22) Date of filing: 10.03.2000
(51) Int. Cl.: A61B 5/00, A61J 7/04

(54) **Method for improving patient compliance with a medical program**

(30) Priority: 11.03.1999 US 266647
(71) Applicant: Brown, Eric W., Newport Beach, California 92660 (US)
(72) Inventor: Brown, Eric W., Newport Beach, California 92660 (US)
(74) Representative: McCarthy, Denis Alexis

(57) **Abstract**

A medical system method comprises steps in using portable medication dispensing and data taking devices for use by an out-patient, and which communicate with a base station, typically within the patients' home. The base station communicates periodically with a remote control station which downloads a medical schedule to the base station and thereby to the portable devices, and also receives usage and medical state information from the patient through the portable devices and the base station. A transmission repetition protocol is used which provides a high degree of confidence that patient information will be received by the remote station. The remote station monitors patient activities and status and provides reports as well as refill requests when supplies are low. Presentations to the patient are provided by Internet or other simplified technique including showing history graphics while describing important features and recommending purchases of supplies and medications.

## Description

### BACKGROUND OF THE INVENTION

### FIELD OF THE INVENTION:

This invention relates generally to the monitoring and control of medical care data by non-medically trained individuals, and more particularly to an automated method of collecting and using patient care data for assuring a high level of healthcare vigilance with feedback to the patient and the patient's caregivers, and with provision for professional medical advice including the selection of offered medications and other medical supplies for sale..

### DESCRIPTION OF RELATED ART:

The following art defines the present state of this field:

Kutzik et al, U.S. 5,692,215 describes a system that provides for monitoring a user in a user living area. The system includes a system controller and an activity detection subsystem. The activity detection subsystem monitors a daily living activity of the user and provides information representative of the daily living activity to the system controller. The system controller includes a control circuit which generates a control signal in response to the daily living activity information obtained by the activity detection subsystem. Control information from the system controller is applied by way of a control information communication channel both to the activity detection subsystem and to a remote monitoring site. The activity detection subsystem may be system for determining the movement of the user around the home, medication compliance by the user, problems with usage for stoves or other potentially dangerous appliances, and selected auxiliary appliances.

Lipscher, U.S. 4,082,084 describes a portable diagnostic device, particularly for medical field-examinations comprising a case-like housing in which replaceable electronic examining units are arranged serving for the examination of different physiological functions and/or conditions. The housing comprises an electronic power supply feeding each of the electronic examining units, a common display receiving the output signals of the examining units and electrical connectors providing electrical connections between the housing and each of the examining units. The inner room of the housing is divided into two separate parts, the first of which is arranged in a modular system and accommodates slide-in examining units, while the second part serves to accommodate the accessory means required for the examinations.

Fu, U.S.4,803,625 provides for a personal health monitor which includes sensors for measuring patient weight, temperature, blood pressure, and ECG waveform. The monitor is coupled to a central unit via modems and includes a computer which is programmed to prompt a patient to take prescribed medication at prescribed times, to use the sensor to measure prescribed health parameters, and to supply answers to selected questions. Medication compliance information, test results, and patient answers are compiled in a composite log which is automatically transmitted to the central unit. The computer is also programmed automatically to disconnect the monitor from an alternating current power source and to rely on internal battery power during certain periods of patient monitor interaction, such as during use of the ECG module. In this way, danger to the patient and complexity of the ECG module are minimized. The computer is also programmed to compare measured test information with predetermined expected values, and in the event of a discrepancy, to collect additional information from the patient to assist trained personnel at the central unit in interpreting the composite log. The computer is also programmed to alert the central unit promptly in the event one or more measured parameters falls outside of a prescribed normal range. The normal range for a given parameter is made to vary in accordance with the measured value of one or more other parameters in order to reduce the incidence of false alarms.

Bornn et al., U.S. 4,827,943 provides a link between the caregiver and the subject being monitored which utilizes an intermediate base station with redundant signal paths between the base station and the caregiver. The caregiver wears a unit which receives signals from the base station. Signals from the base station provide information about the subject being monitored and provides signals for use in determining whether the caregiver remains within the range of the base station. The unit worn by the subject being monitored can include diagnostic circuitry for evaluating signals received from sensors to transmit an alarm signal to the base stations when the subject being monitored is in need of assistance. A range monitoring system is provided which alerts the subject being monitored as well as the caregiver whenever the subject being monitored moves outside the range of the base station.

Kaufman, U.S. 4,933,873 describes an interactive patient assistance device which houses both pre-selected doses of medication and a physical testing device. Both medication and the testing device are normally retained within separate compartments within the device away from access by the patient. The device keeps track of medication and diagnostic testing schedules. The device is also capable of receiving and interpreting verbal commands of the patient. The device makes a pre-selected dose of medication available to the patient in response to either the medication schedule or the receipt of a verbal command by the patient. Likewise, the testing device is made available to the patient in response either to the testing schedule or the receipt of a verbal command from the patient.

Treatch, U.S. 5,007,429 describes a user interface for directing the programming of operating parameters for patient blood pressure testing into and downloading blood pressure data from ambulatory patient blood pressure monitoring units. The user interface operates on a system comprising a plurality of microprocessor based, ambulatory blood pressure measuring patient units, an office control unit, and a data processing center, typically accessed over telephone lines. An office control unit is used to program patient units with test regimens for specific patients. The control units are also used to download data from the patient units and to transfer the data, along with patient identifying data, to the central data processing facility. The office control unit includes local memory which stores various interface routines, a microprocessor for executing the routines, a 12-character keypad allowing input of integers and a display for displaying prompts to the user. Upon initial power up of the control unit, and operator using the control unit is prompted through a start up sequence and a menu selection sequence to carry out the desired functions of the system. All selections are made, and all operating parameters are entered, through a telephone like keypad. The display indicates to the user which parameter entry of which is called for and which menu items are available for selection. During transfer of data to the central processing facility, additional prompts may be given to the operator by voice over the telephone handset.

Blomquist, U.S. 5,338,157 describes an invention relating to systems and methods for communicating with ambulatory medical devices, such as drug delivery devices, both locally and remotely. In one embodiment, a caregiver drug pump communicates with a remote patient drug pump for data gathering, trouble shooting, and operational program changes. The caregiver drug pump is at least substantially identical in configuration to the patient drug pump. The caregiver drub pump transmits caregiver key input signals to the remote patient drug pump. The patient drug pump receives the key input signals, accesses a desired program, and transmits information for display on the display of the caregiver drug pump. In another embodiment, a computer is provided for communicating locally and/or remotely with a drug pump. The computer may include a display with an image of a pump. The computer may be operated through the use of a mouse or touch screen with respect to the image of the pump, to simulate use of the pump while using the personal computer. The computer may also be used as a training aid for training a caregiver and/or patient how to use the drug pump.

Maestre, U.S. 5,347,453 describes a portable programmable medication device for aiding in the administration of medication or pharmaceuticals in accordance with a prescribed medication dosage schedule. In a first illustrative embodiment, the programmable medication alarm device is manually programmed with data representative of a prescribed medication dosage schedule specifying a prescribed administration time, dosage amount, administration route, and medication instructions for each medication dosage to be administered to the patient. In response to the time occurrence of each programmed administration time, and audible dosage alarm signal is generated and graphical representations of the prescribed administration time, dosage amount, administration route and medication instructions are visually displayed in predefined visual display fields. In a second illustrative embodiment, the portable medication alarm device is programmed by loading the prescribed dosage schedule data from a computer system, into the memory of the medication alarm device, using an automated data communication process. Also disclosed is a medication container holder which attaches the programmed medication alarm device to a conventional medication container, such as an eye-drop dispenser bottle, nasal-spray canister or pill bottle, without interfering with the operation thereof.

Stutman, U.S. 5,416,695 describes a medical alert system which enables an authorized user, such as a doctor, to remotely set selection and limit parameters pertaining to specific medical and geodetic information of an ambulatory patient and thereater received updates of that information over a wireless communication network when the parameters have been met. A telemetry device attached to the patient provides an inbound stream of medical and geodetic information to a host computer, which is configured to exact selected portions of that information in response to the parameters provided by a remote processing device via a communications network. Upon completion of the latter process, the host computer transfers the extracted information to the remote processing device over the network, thereby informing the doctor of a medical situation.

Maestre, U.S. 5,495,961 describes a portable programmable medication alarm device for aiding in the administration of medication or pharmaceuticals in accordance with a prescribed medication dosage schedule. In a first illustrative embodiment, the programmable medication alarm device is manually programmed with data representative of a prescribed medication dosage schedule specifying a prescribed administration time, dosage amount, administration route, and medication instructions for each medication dosage to be administered to the patient. In response to the timed occurrence of each programmed administration time, an audible dosage alarm signal is generated and graphical representations of the prescribed administration time, dosage amount, administration route, and medication instructions are visually displayed in predefined visual display fields. In a second illustrative embodiment, the portable medication alarm device is programmed by loading the prescribed dosage schedule data from a computer system, into the memory of the medication alarm device, using an automated data communication process. Also disclosed is a medication container holder which attaches the programmed medication alarm device to a conventional medication container, such as an eye-drop dispenser bottle, nasal-spray canister or pill bottle, without interfering with the operation thereof.

Vasko, U.S. 5,573,506 describes a remotely programmable infusion pump with interactive voice response via touch-tone phone (i.e., voice mail system in IV pump). The remotely programmable infusion system also comprises a voice storage unit for storing the voice signal. The remotely programmable infusion system further comprises a processor, coupled to the remote communication port, to the voice storage unit, and to the memory, for accessing the voice signal from the voice storage unit and the programmable protocol from the memory, and for processing the programmable protocol in response to receiving the remote programming signal.

Kurtenbach, U.S. 5,582,323 describes a medication dispensing and monitoring system of a present invention includes a housing containing a plurality of pill dispensing compartments for dispensing medication to a patient at a desired time. The invention is programmed to dispense medication at the desired time and activates alarms if the proper procedure is not completed. The invention also contacts the emergency personnel through phone lines and initiates two-way hands free communication between the patient and emergency personnel.

The invention further includes a pendent transmitter worn by the patient to contact emergency personnel.

Hultman, U.S. 5,582,593 describes an ambulatory medication delivery system which includes an ambulatory pump unit having a computer control linear motor pump for pumping predetermined volumes of fluid in accordance with a programmed delivery schedule which may be altered through communication with a remote monitoring location via a telephone data access line or via radio frequency communication. A clinician communication unit and a patient communication unit receive and send information to the ambulatory pump unit and also communicate via a telephone data modem access to the computer at a remote monitoring location at which trained health personnel can monitor a number of patient locations and alter or change medication delivery profiles as required.

Tacklind, U.S. 5,704,366 describes a system for monitoring and reporting medical information includes a stand-alone monitor for storing data records comprising measured values and time stamps and for transmitting the records to a remote reporting unit over a communication system. The remote reporting unit includes a relational data base that is updated when records are downloaded from the monitor; a report generator for generating chronological graphs of the measured values for a particular patient; and a report transmitting unit for transmitting reports to a requesting health care provider.

Ridgeway, U.S. 5,710,551 describes a system for the remote monitoring of in-home self-medication to assure compliance with prescribed dosage schedules. The system comprises at least one subscriber home medication station which interfaces with a communications link and a remote central monitoring station also interfaced with the link and operative to receive and analyze messages transmitted by the home medication station. The preferred home medication station embodiment transmits messages to the central station over the communications link each time the home medication station is accessed for a dosage of medication. Central station computer means verify receipt of such signals within each subscriber's uniquely scheduled dosage time windows, and alert an operator to take appropriate action if a dosage schedule error is detected. Alternative home medication station embodiments utilize a built-in programmable timer module to verify accessing of medication within a subscriber's uniquely scheduled dosage time windows, and to initiate transmission of alarm signals to the central station over the communications link if dosage schedule errors are detected by the timer module. All embodiments provide subscribers with help-button means to initiate transmission of alarm messages to the central station over the communications link in event of adverse reaction to medication, or other emergencies. Since the central station will be alerted if any scheduled dosage is missed, no emergency rendering a subscriber unable to press a help-button or call for help can go undetected longer than the maximum time between consecutively scheduled dosages.

Tacklind, U.S. 5,752,709 describes a system for monitoring and reporting medical information includes a stand-alone monitor for storing data records comprising measured values and time stamps and for transmitting the records to a remote reporting unit over a communication system. The remote reporting unit includes a relational data base that is updated when records are downloaded from the monitor; a report generator for generating chronological graphs of the measured values for a particular patient; and a report transmitting unit for transmitting reports to a requesting health care provider.

Stoop, U.S. 5,767,791 describes a two-way telemetry system which displays and monitors physiologic and other patient data of multiple, remotely located patients at a central location. The system comprises multiple battery-powered remote telemeters, each of which is worn by a respective patient, and a central station which receives, displays, and monitors the patient data received from the remote telemeter. The telemeters communicate with the central station using a two-way TDMA protocol which permits the time sharing of timeslots, and which uses a contention slot to permit telemeters to transmit service requests to the central station. Two-way special diversity is provided using only one antenna and one transceiver on each remote telemeter. The remote telemeters include circuitry for turning off the active transceiver components thereof when not in use (to conserve battery power), and include circuitry for performing a rapid, low-power frequency lock cycle upon power-up.

The system has multiple modes of operation, including a frequency hopping (spread spectrum) mode and a fixed frequency mode, both of which preferably make use of the 902-928 MHz ISM band. Patient locators are provided to allow the clinician to track the location of each patient.

Russo, U.S. 5,807,336 describes a medical apparatus that is provided with a programmable medical device disposed at a first room location and a remote monitor and/or controller disposed at a second room location. The programmable medical device is used to administer a medical treatment to a patient, and the remote monitor/controller may be used to monitor the operation of the medical device, control the operation of the medical device, and/or transfer data from the medical device to the remote monitor/controller. The apparatus may allow voice communication between the remote monitor/controller and the patient who is receiving treatment via the medical device while the medical device is being monitored and/or controlled from the remote location. The remote monitor/controller may also include means for determining the type of medical device to which it is connected.

Brudny et al., U.S. 5,810,747 describes an interactive intervention training system used for monitoring a patient suffering from neurological disorders of movement or a subject seeking to improve skill performance and assisting their training. A patient (or trainee) station is used in interactive training. The patient (or trainee) station includes a computer. A supervisor station is used by, for example, a medical or other professional. The patient (or trainee) station and the supervisor station can communicate with each other, for example, over the Internet or over LAN. The patient (or trainee) station may be located remotely or locally with respect to the supervisor station. Sensors collect physiologic information and physical information from the patient or subject while the patient or subject is undergoing training. This information is provided to the supervisor station. It may be summarized and displayed to the patient/subject and/or the supervisor. The patient/subject and the supervisor can communicate with each other, for example, via video, in real time. An expert system and neural network determine a goal to be achieved during training. There may be more than one patient (or trainee) station, thus allowing the supervisor to supervise a number of patients/subjects concurrently.

The prior art teaches the use of electronic measuring and monitoring of patients. However, the prior art does not teach that an automated medication and monitoring system with feedback can be used with such a method that data completeness and accuracy is assured through a novel signal repetition protocol. Also, the prior art does not teach that reporting of health results to the patient may be done in an interactive manner coupled with professional medical advisers helping, through a novel remote communication protocol, to direct optimum patient behavior, including offering of medical supplies and medications associated with an ongoing management program. The present invention fulfills these needs and provides further related advantages as described in the following summary.

### SUMMARY OF THE INVENTION

The present invention teaches certain benefits in data handling methods which give rise to the objectives described below.

The present invention provides a home care medical data logging, communication, and reporting method that has not been described in the prior art.

A primary objective of the present invention is to provide such an method having advantages not taught by the prior art and which are capable of accurate medical data recording especially for a non-compliant patient.

Another objective is to provide such a method capable of assuring the transmission of accurate medical data although a patient is not within data transmission range of a data receiving station for significant periods of time.

Yet another objective is to provide such a method having means of correlating and reporting health and behavioral status on an interactive basis so as to improve compliance by a reluctant patient.

A further objective is to provide such a method enabled for offering and motivating a patient to purchase supplies and medications in accordance with a medical care program.

Other features and advantages of the present invention will become apparent from the following more detailed description, taken in conjunction with the accompanying drawings, which illustrate, by way of example, the principles of the invention.

### BRIEF DESCRIPTION OF THE DRAWING

The accompanying drawings illustrate the present invention. In such drawing:
FIGURE 1 is a schematic block diagram showing the relationship between major elements of the invention;
FIGURE 2 is a schematic block diagram of a typical dispensing device thereof;
FIGURE 3 is a typical printed report of the invention illustrating medication dosing compliance correlated to patient status;
FIGURE 4 is a typical monitor screen display report of the invention illustrating dosing compliance correlated to patient status;
FIGURE 5 is a typical monitor screen display report of the invention illustrating oral medication compliance;
FIGURE 6 is a typical monitor screen display report of the invention illustrating daily sugar level variation for a diabetes patient and showing the measurement device therefor;
FIGURE 7 is a typical monitor screen display of the invention for inputting patient care orders for, in the present case, medication instructions; and
FIGURE 8 is a typical monitor screen display of the invention illustrating a method of delivering health or behavior information with a nurse, clinician or other helper providing audio and video transmission of explanations, information, feedback, suggestions, instruction on ordering medication refills and supplies, behavior modification and control and other invention pertinent communications.

### DETAILED DESCRIPTION OF THE INVENTION

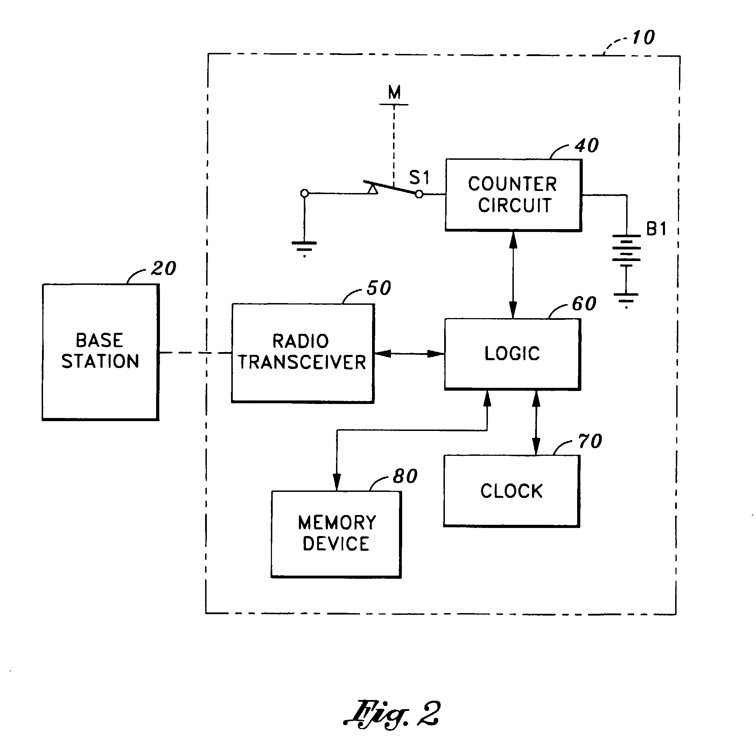

The above described drawing figures illustrate the invention, a system including apparatus and method, for improving patient compliance with a medical program. The medical program may include medication dispensing elements as well as medical state sensing elements or both. The invention is used to monitor and modify medical care and especially patient behavior patterns associated with medication dispensing, medical state sensing and patient behavior with respect to acquisition and maintenance of medical treatment related supplies, wherein the apparatus comprises: a portable medical device means 10 providing, in one embodiment, a medication storing means such as a pill bottle, a medication accessing means such as the cap to the pill bottle, a dispensed medication counting means such as an electronic flip-flop counting circuit 40 with a simple numerical indexer, preferably powered by a battery Bl, in communication with the pill bottle cap by any electromechanical, magnetic or other physical element known to one of skill in the art and represented by letter "M" in Figure 2, such that when the cap is removed from the pill bottle, a switch S1 is made such that the circuit counting means indexes to a next higher numerical value, and a medical device wave energy communicating means such as a radio transceiver 50 is activated. The portable medical device means 10 may include a single dispensing and counting unit or multiple units, and further may include a single counting unit for multiple dispensed items, or multiple counting units (for redundancy) for a single dispensed item; the dispensed medication counting means being enabled for sequencing the counting circuit 40 each time the medication accessing means is manipulated, for gaining access to the medication storing means, the device means 10 being further enabled by a logic circuit 60 for establishing a data set comprising, for example, the current numerical value of the counting circuit 40 and a corresponding time and date of the sequencing of the counting circuit 40 as through the use of a clock circuit 70, the wave energy communicating means 50 being enabled by a control program in the logic circuit 60, for example, for repetitively transmitting, as for example, every 30 minutes, a wave energy signal corresponding to each newly established data set during a selected period of transmission time, as for example 72 hours, the transmission time period being chosen to assure a selected probability of the receipt of said wave energy signal by a base station 20 which is remotely located with respect to the medical device means 10 but which is within practical wave energy receiving range frequently. Because the transmission range of the transceiver is limited by FCC rules, it is conceivable, and quite common for the portable medical device means 10 to be carried by the patient to a location that is not within the range of the base station 20. This would happen on a regular basis if, for instance, the patient was in the habit of taking a walk in the park between 2 PM and 4 PM each day and carries his pill bottle so as to be able to take his 3 PM pills. This dosage would be counted when the pill bottle cap is removed, but could not be received immediately at the base station 20 since the pill bottle would be out of range at that time.

In another embodiment the invention comprises: the portable medical device means 10 providing a medical state sensing means and the medical device wave energy communicating means 50 of the first embodiment; the medical state sensing means is enabled for sensing a medical state variable, such as body temperature, body weight, blood pressure level, or blood glucose level each time the sensing means, i.e., thermometer, weight scale, sphygmomanometer or blood glucose measurement system, respectively, is manipulated in accordance with any well known medical state variable manipulation protocol, i.e., common to any medical examination office or clinic, and further enabled, by a simple mechanical arrangement "M" such as the use of a sensor for sequencing the electronic counter 40, as described above, each time the sensing means is manipulated and for establishing a data set comprising a value of the measured medical state, a numerical value of the counter 40 and a corresponding time and date of the measurement from the clock circuit 70, the wave energy communicating means 50 being enabled, as above, for repetitively transmitting a wave energy signal corresponding to each newly established data set for a selected period of transmission time, the transmission time period, again, being chosen to assure a selected probability of the receipt of said wave energy signal by the base station 20 as previously described.

In either embodiment, the invention further preferably comprises a human responsive signaling means, such as an audible alarm or a vibration generating device (not shown), enabled in accordance with the patient's medication and state measurement schedule, referred to below as the "medical schedule," to alert the patient when medication dosing or medical state sensing is required. Such audible and vibration alarms, i.e., attention demanding techniques, are well known in the personal pager industry.

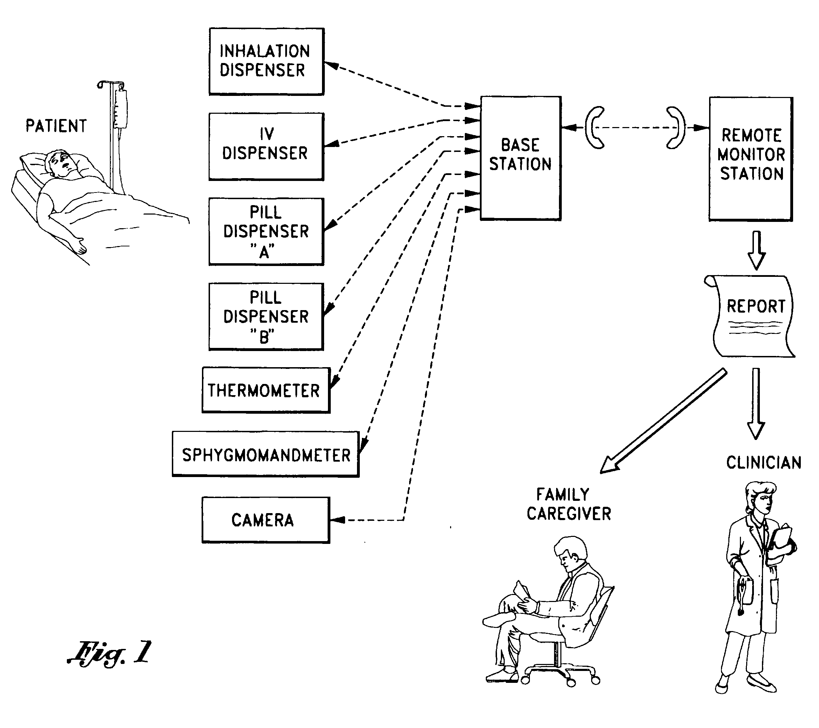

Preferably, the base station 20 comprises a base station wave energy communicating means, such as a radio transceiver 50 similar or identical to that of the medical device means 10, and is preferably physically located in the patient's home for example, for enabling reception of at least one repetition of the wave energy signal of the medical device wave energy communicating means 50 and also for transmitting the medical schedule to the medical device means 10, the base station 20 further providing a primary, two-way communicating means 90 shown in Figure 1, such as a telephony or other communication means, enabled by logic circuits and software programming, as is well known in systems engineering, for automatically transmitting the data set values of the medical device means 10, as received via the wave energy signal, in accordance with a selected communicating schedule. That is to say, the information received at the base station 20 from one or more of the portable medical device means 10 is transmitted via the two-way communicating means 90 to a remote monitoring station 30, the remote monitoring station 30 further comprising a computation means, such as a general purpose computer, for computing summary information of interest to medical professionals and caregivers and the patient him/herself, such as a correlation analysis between the received data set values and a selected medical state parameter that is being monitored, the remote monitoring station 30 comprising further enablement, by modem or other means, for transmitting information of interest to the primary, two-way communicating means via the secondary, two-way communicating means 90 and for transmitting the selected medical schedule, usually a schedule defined by a primary medical care giver such as the patient's medical doctor. Preferably the base station 20 receives a unique identification code "PB187" representing "pill bottle 187" or "BPM456" representing blood pressure monitor 456," and "1030A-120198" representing a time-date stamp corresponding to, 10:30 AM on Dec. 1, 1998, and, in the case of a medical state measurement, additionally a measured value code such as "125/80" representing blood pressure levels at the time and date of the measurement. Such a transmission would therefore be coded as "BPM456.1030A-120198.125/80" and may be transmitted in binary coded decimal form or converted to digital form prior to transmission. Other transmissions from the various medical device means 10 may include a low battery status, out of medication, or other supply, "active," "standby," or "inoperative" status, etc. along with the time/date identification. The software protocol used in the medical device means 10 or in the base station 20, or both, is preferably configured for identification of an emergency state, as when blood pressure or glucose level are at a dangerous level. In this case the medical device means 10 will immediately set an audible or other alarm to request a repeat of the measurement by the patient or his caregiver. In the following, and clearly throughout this description and the claims that follow, the use of "patient" should be recognized as including the actual patient, or his immediate caregiver such as a family member or a care provider such as a practical nurse or a medical worker. It is intended that preferably the patient will be active with respect to the present invention, at least in part, but if the patient is not active, a caregiver acts for the patient and as such, the caregiver must be at least frequently in close proximity to the actual patient.

The base station 20 is configured to immediately send an alarm message to the remote monitoring station 30 for immediate authoritative supervision and decision making, etc. The means for accomplishing these results, i.e., enablement, is most efficaciously completed through software logic programs controlled by microprocessor means within the medical device means 10 and the base station 20 means. Such enablement is considered to be within the ability of those of average skill in the art, and are therefore not defined here in further detail.

Clearly, the portable medical device means 10 may be any one or more of the well known dispensing devices in use in pharmacy and medicine such as an inhalant dispenser, an intravenous dispensing system, an oral or anal medication dispenser, a topical formulation dispenser, etc., for dispensing a selected dosage of a medicinal inhalant, intravenous medication, medicaments formulated as pills, capsules, caplets or other forms of solid medications or liquid or other forms of internal medications, as well as topical formulations. In each case the medication accessing means, such as a bottle cap, a dispensing tube winder, an inhalant pump, or a IV flow meter, is adapted by any simple well known sensing arrangement to trigger the counting means while simultaneously dispensing a previously selected dosage of the medication. Alternately, the dispensed amount may be controlled by the patient or the on-site caregiver.

In the preferred embodiment the medical device wave energy communicating means 50 of the medical device means 10 is adapted for accomplishing certain inventive methods, through any common or well known data manipulation process for receiving and implementing the medical schedule as transmitted from the base station 20, a simple matter of data transmission and receival. In this manner, the medication or state sensing schedule is remotely set-up within a memory device or circuit 80 of the medical device means 10, and may also be changed at any time. Also, the remote monitoring station 30 is adapted by these methods, i.e., simple tracking protocols, for composing a refill request message, at appropriate times when its calculations show that supplies are low, and for transmitting the refill request message to the base station 20 to alert the patient by, for instance, illuminating a lamp or presenting a written message on an LCD, or similar display on the base station, and, or on the medical device means 10.

Preferably, the methods of the present invention are adapted for composing graphical summary reports showing, for instance, when each medication was, in fact, taken and when each medical measurement was, in fact, completed. Such a history of events may be for a week, month or longer so as to enable the patient to see trends. The portable medical device means 10 of the invention is enabled, by memory device 80, for storing a list of all said data sets established over the selected period of transmission time, e.g., 72 hours, the list being transmitted repetitively at a selected frequency of transmission, e.g., every 30 minutes, so as to improve, through transmission redundancy, the probability of receipt, by the base station 20, of all data sets established by the medical device means 10. In the preferred embodiment, each of the data sets is paired with a check-sum and with a CRC, the base station 20 providing check-sum and CRC error-reducing protocol operating on the data sets as received for providing error reduction. These error checking protocols are well known, but have not been previously used in the field of the present application, and are certainly critically important in the present use.

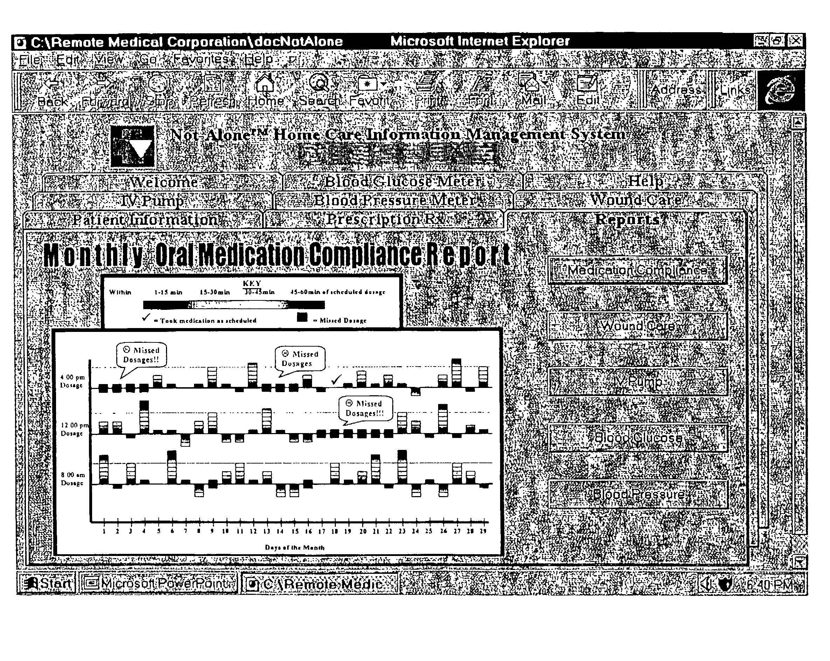

In use, the patient notifies the remote monitoring center as to the master medical schedule for medications and/or medical state measurements required. This may be accomplished by telephone, fax, written communication, or internet screen, the latter being illustrated in Figure 7. A database is established at the remote monitoring center for the patient. The master medical schedule information is downloaded to the base station 20 through the primary and the secondary communicating means, generally via telephony. The base station 20 then transmits an appropriate portion of the master medical schedule to each of the medical device means 10 which will be used by the patient. Each of the medical device means 10 then alerts the patient when a medication or a measurement should be taken. The medical device means 10 continues to remind the patient that such event is required until it is accomplished. The fulfillment of each event is recorded as described above and entered into the event list. The entire list is transmitted to the base station 20, as defined above, every selected period, such as every 30 minutes over a time duration of 72 hours for example or other selected duration. Each time the list is received by the base station 20 it is compared with the resident list at the base station 20 and any blanks in the resident list are filled-in from the newly received list. The duplicate entries are then discarded so that the resultant list is complete and non-redundant.

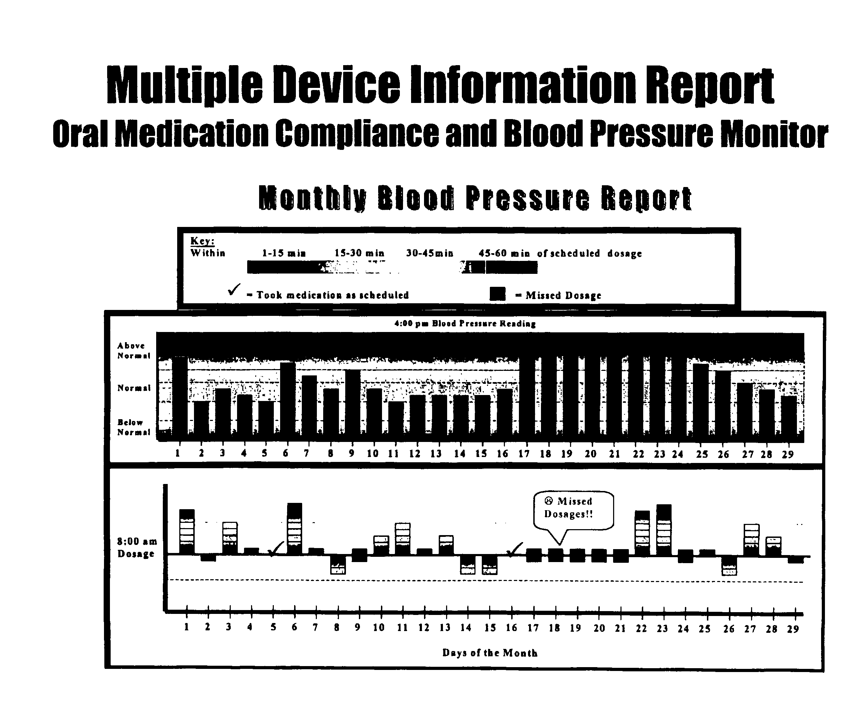

The invention further provides a reporting function which is illustrated in Figures 3-6 and 8. Figure 3 illustrates a correlation report which may be used to highlight the importance of behavioral compliance with a medication schedule for maintenance of a health parameter, such as blood pressure level or blood glucose level. Individual data reports are well-known in the medical industry, but graphical reports of patient behavior versus one or more health statistics, as defined by a chart, such as a bar chart, is novel and considered distinct from the prior art.

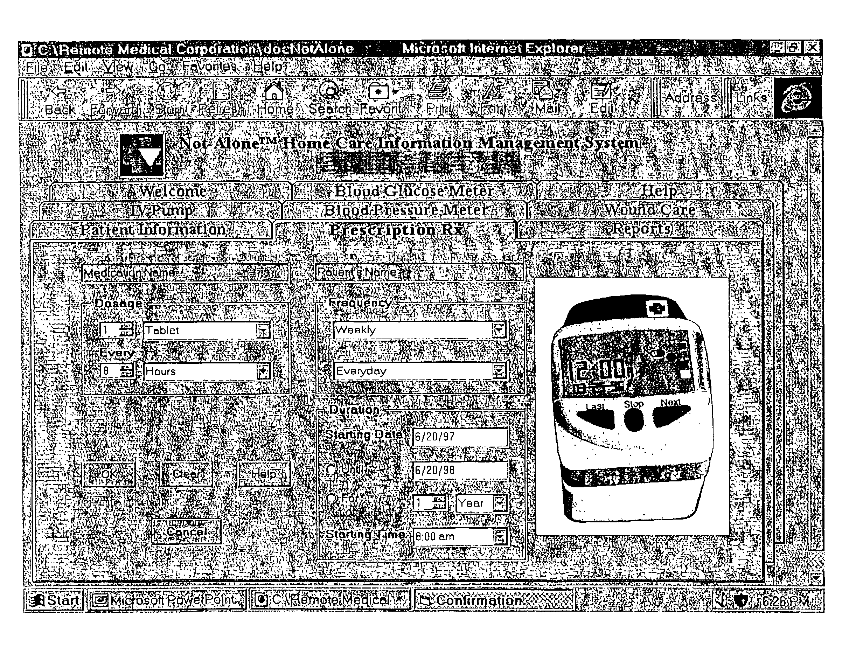

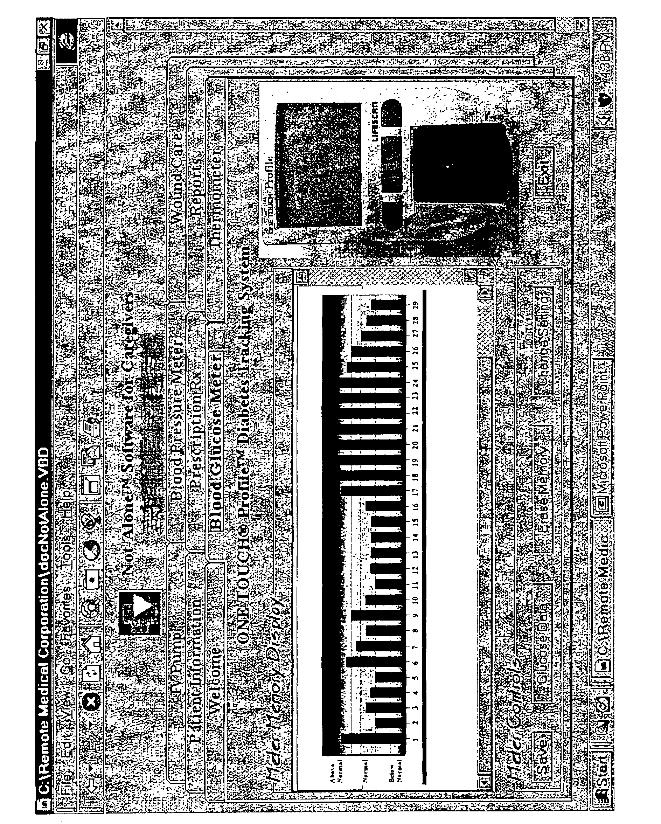

Once the reports are generated by the remote monitoring station 30, they are delivered to the patient, to enable action to be taken. Delivery modalities include hardcopy paper reports sent by mail delivery, faxed copies, or internet web-page delivery. The internet web-page delivery is preferred because it economically allows the use of color graphics. Color might be used in Figures 4 and 5, for example, to highlight deviations from the scheduled dosage time such that missed dosages or delayed dosages are shown in red and on-time dosages are shown in green. Highlighting good and bad behavior patterns to the patient is functional for establishing improved behavior.

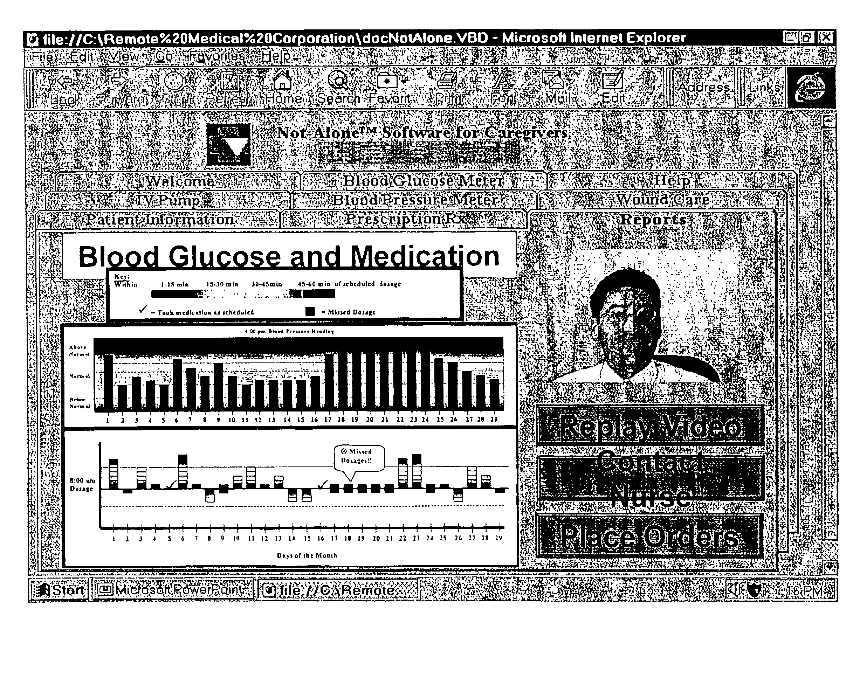

Figure 4 illustrates the use of reports similar to Figure 3, but in a web-page format. Often patients become confused when they must use many different of the medical devices means. Figure 6 illustrates how information can be more readily associated with a specific device to enhance patient recognition and understanding, i.e., the inclusion of a picture of the device the patient is using. Figure 6 also shows controls (screen buttons) whereby the patient or local caregiver may send commands pertaining to device settings directly to the remote monitoring station 30. The remote monitoring station 30, in turn, is enabled for relaying these control messages to the base station 20 located in the patient's residence. In this manner, the web-page format is used to provide the patient with information and can also be used by a clinician at a remote location to control a patient's instruments, for example, based upon the health data statistics that are compiled.

Figure 7 illustrates how the web-page format can be entirely dedicated to the setup and control of a medical device means 10, such as a medication dispenser. In Figure 7, a clinician at the remote monitoring center or at any remote location can enter dosing related information and instructions and the medical schedule for that medication dispenser.

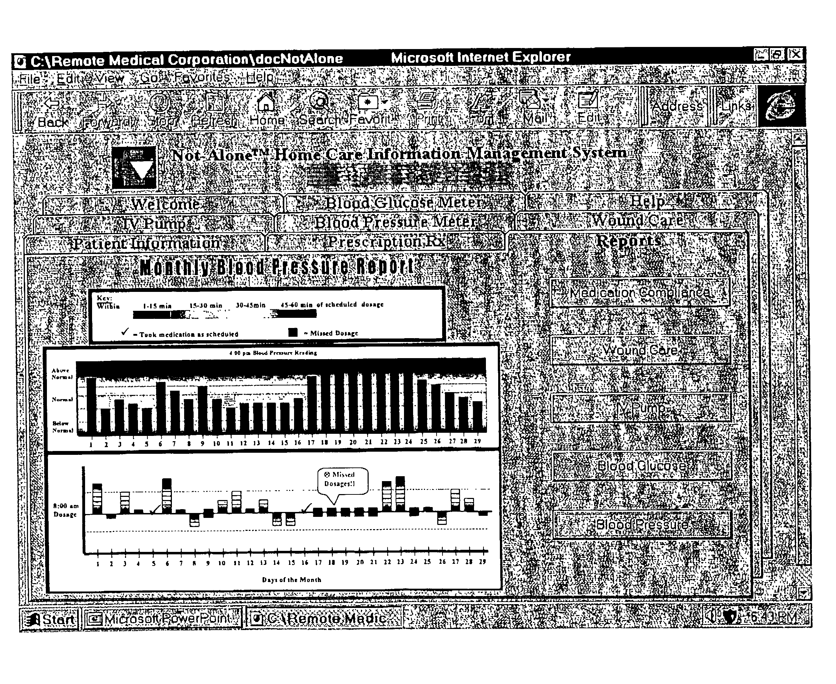

Figure 8 illustrates an advance in the reporting of medical information to patients. The advance includes the correlation of two or more behavior and/or health data graphics which are constructed from actual measurements of the patient's compliance or health statistics. Further, the report includes a human image and related spoken message which is preferably a video message but may be live. This human image is preferably a clinician, such as a nurse, but may also be a doctor or other primary caregiver. As stated, the message may be delivered in real-time, but is preferably delivered in a stored format, such as by e-mail or a stored streaming video, so that the patient may view it or replay it more than once, at his or her convenience. Such replay is facilitated by a dedicated replay button as shown in the figure. The video message report may include elements of encouragement, education, information, reinforcement, or reprimand, as may be needed to modify the behavior of the patient to comply with instructions. Such elements may further include a list of items which the patient may need or want to purchase or otherwise obtain, such as medication refills, information flyers, insurance information, and miscellaneous items such as flowers, greeting cards, etc. for improving the patient's morale.

The reports are preferably interactive so as to enable the patient to easily reply to questions or ask questions via a dedicated contact button. Such a button will, in one embodiment, activate an e-mail reply screen, as is well-known, that may generate text, voice or video image formats for composing a reply message. Further, the report includes purchasing reminders, clues or requests made by the clinician image, such as to remind the patient to purchase refills on medication or medical device supplies, such as blood glucose monitoring test strips, to order home supplies such as flowers, books, videos, music recordings, or other household goods, or to order new equipment such as an additional pill bottle for a new medication. Even further, the report allows the patient to respond to such reminders, clues or requests via a "place order" button, as illustrated. The place order button, depending upon the type of account established with the remote monitoring center, and may immediately fulfill the order suggested by the video image or it may lead to order selection and confirmation screens, as is well-known in the art. The interactive report may further include an invitation to reply to any questions posed by the clinician image, or to pose new questions to the clinician image, or to respond to previous questions posed by the patient to the clinician image. Additionally, the clinician image may suggest that the patient replay the video message to reinforce an understanding of its contents. The use of interactive reports as defined herein is considered novel in the present field, so as to distinguish over the prior art.

The following charts define: the preferred manner in which the video reports (screens) are created, preferred screen content, how the screens are used and preferred follow-up action. Please note the use of B-S representing "base station 20" and M-C representing "remote monitoring station 30."

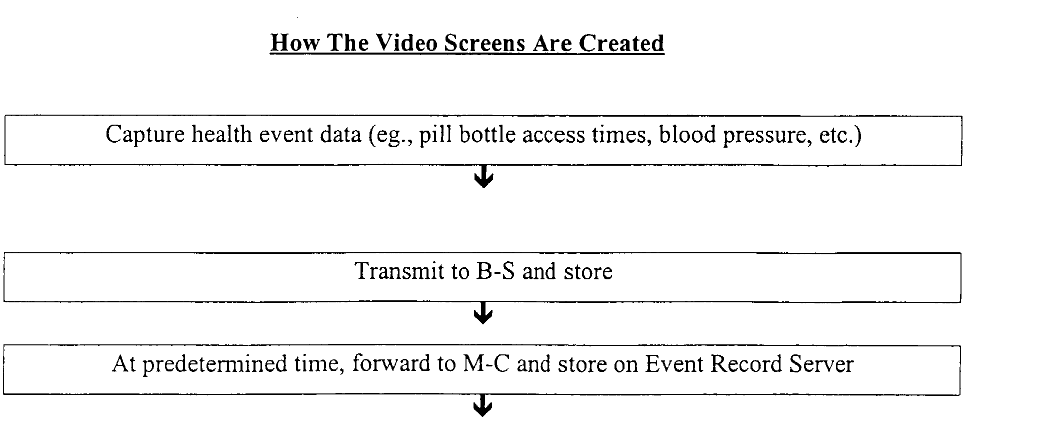

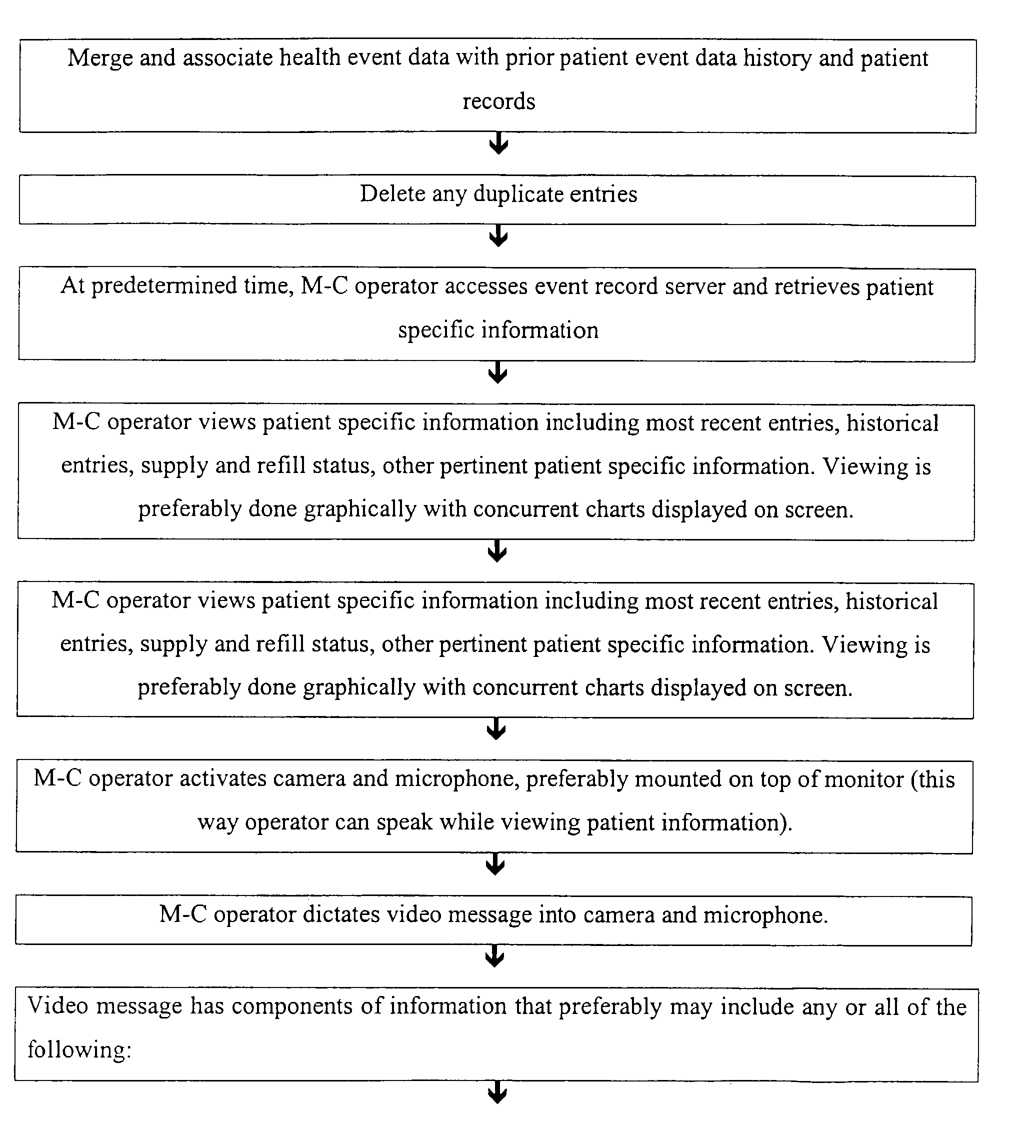

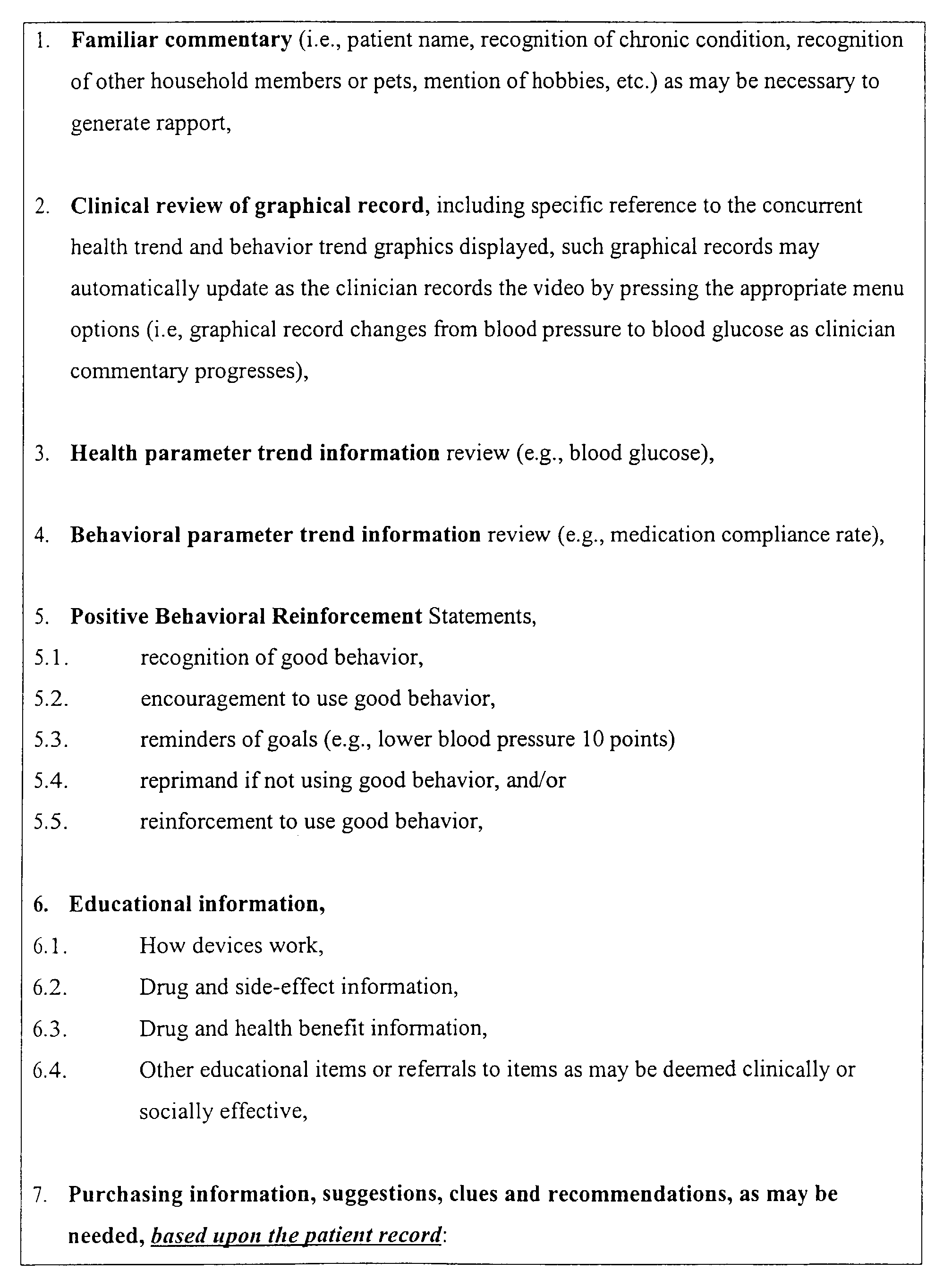

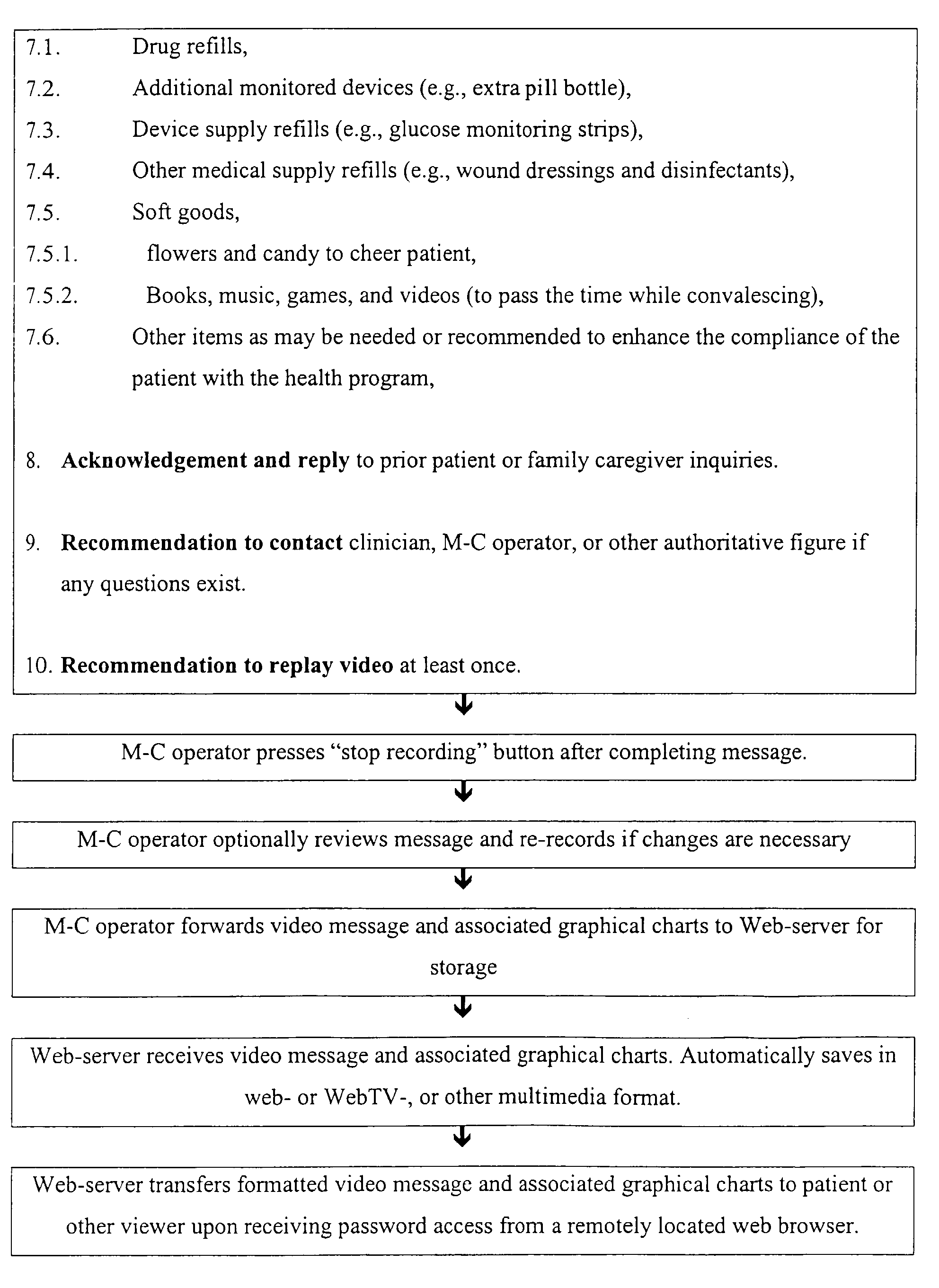

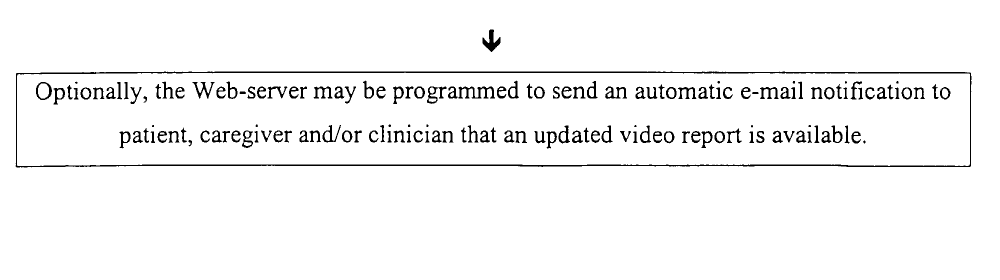

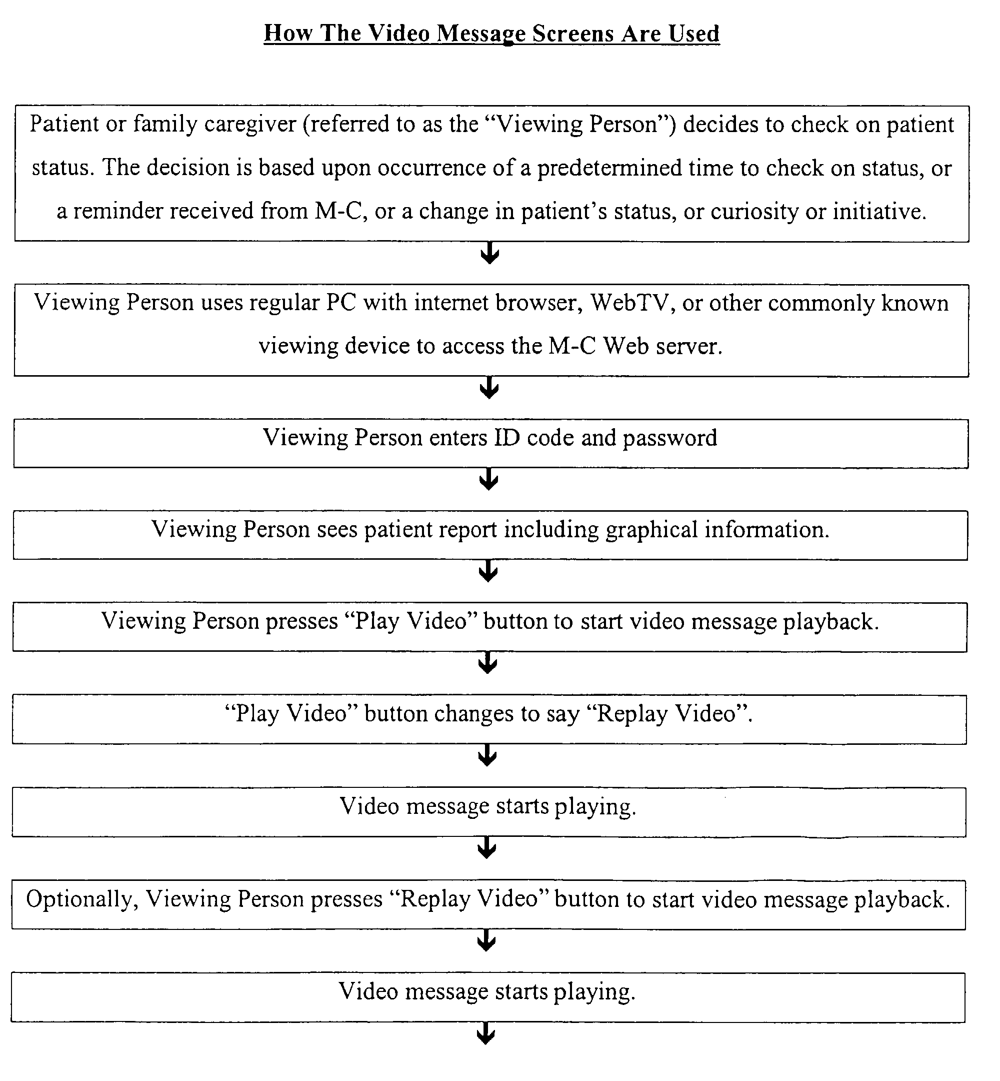

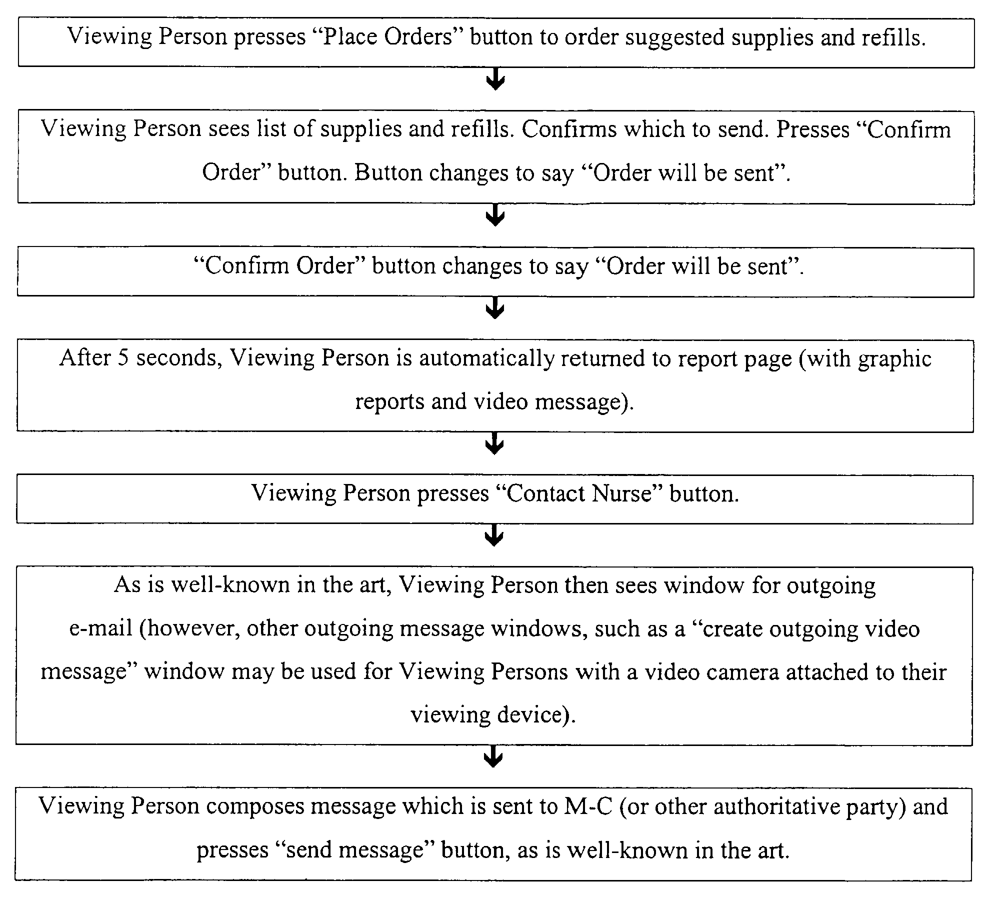

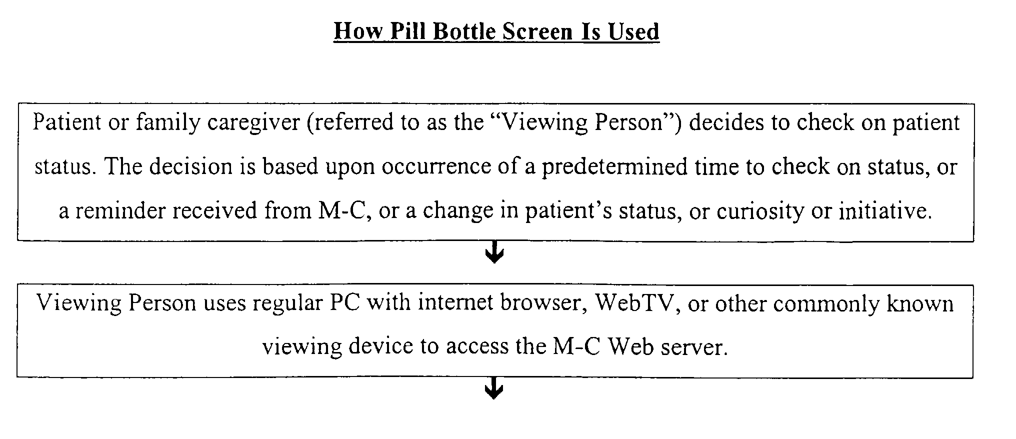

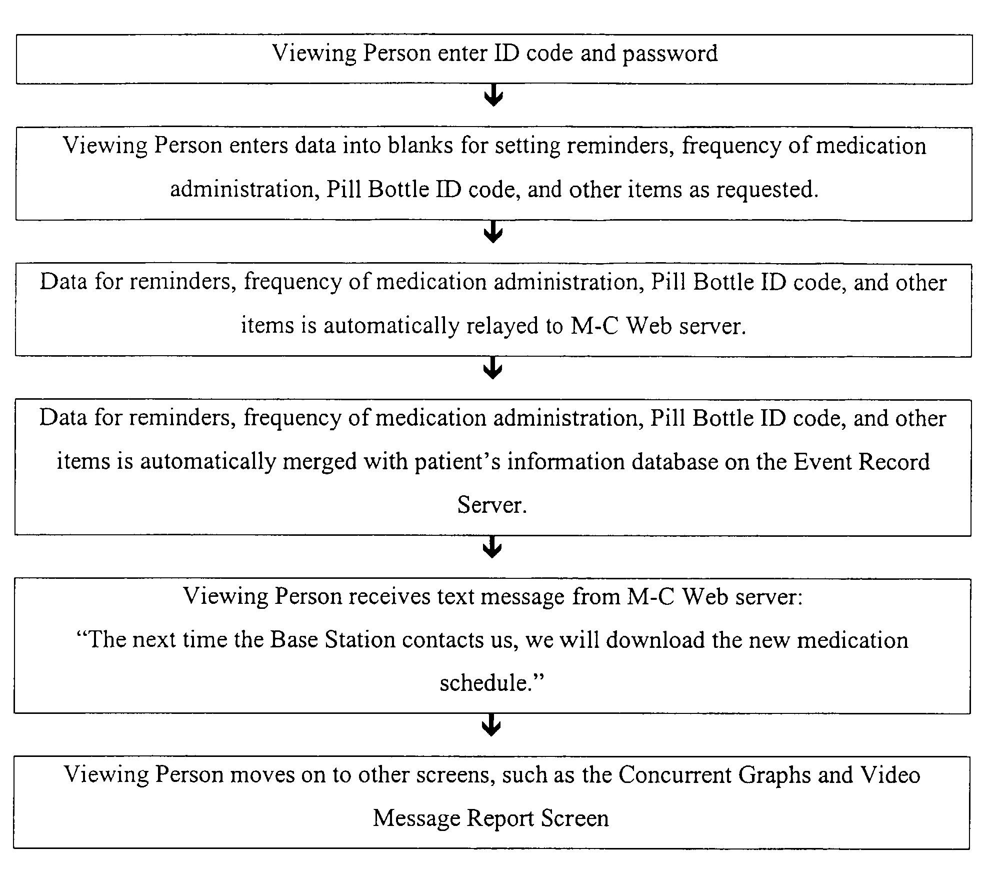

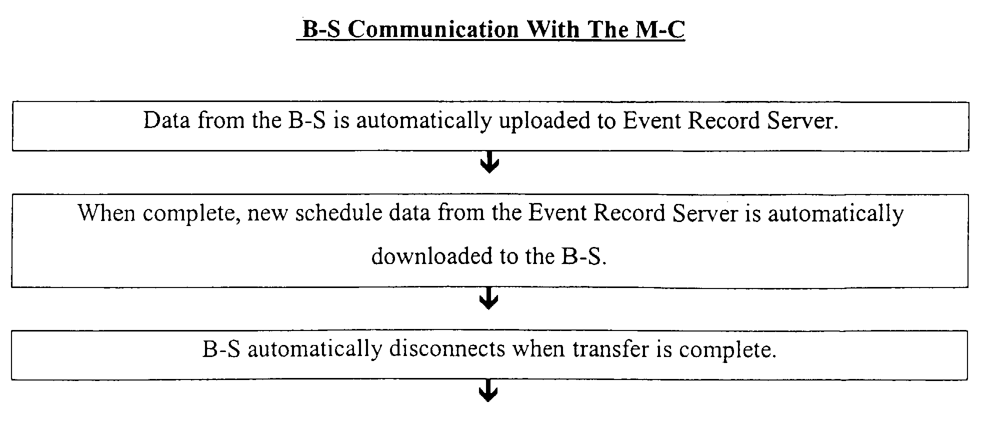

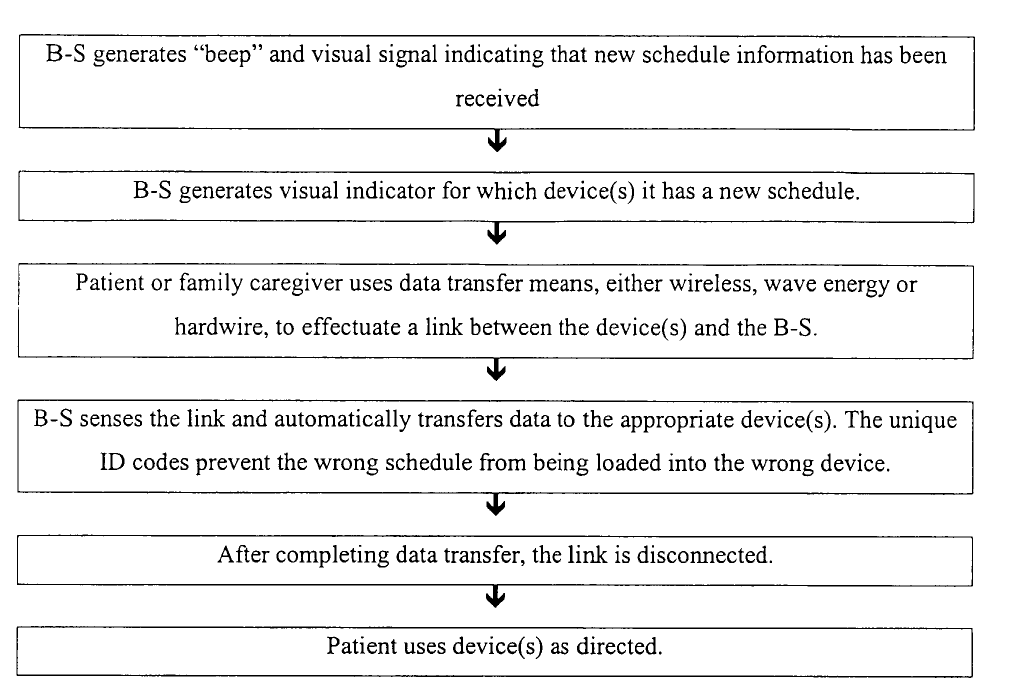

From the diagrams above it is seen that the present invention provides for a method for creating video screens in a medical monitoring and control system comprising the sequenced steps:
a) automatic logging of health event data from dispensing devices and/or medical state measurement devices, into a storage means at the time of such events as enabled by actions of a patient or a patient with the help of a caregiver usually in the patient's home;
b) automatic transmitting of the health event data to a local base station for storage therein, the base station being generally in the patient's home;
c) automatic transmitting of the health event data on a predetermined schedule to a remote monitoring center for storage therein, the center being located outside the patient's home and usually in a distant location, possibly near a hospital or doctor's office;
d) automatic merging of each new transmission of the event data with an existing record of all previously recorded event data from the same base station;
e) deleting duplicate entries of the event data;
f) retrieving the event data as an event data report comprising charts, graphs, etc.;
g) medical personnel evaluating of the event data report;
h) medical personnel recording of comments concerning the event data report, the comments comprising familiar commentary, clinical review of graphical records, health parameter trend information, behavioral parameter trend information, positive behavioral reinforcement, educational information, purchasing information, suggestions, clues and recommendations, acknowledgements and replies, and recommendations;
i) transmitting or sending the recorded comments of step (h) to storage for saving in multimedia format; and
j) sending the recorded comments of step (i) to the caregiver upon his/her demand.
k) caregiver viewing of the recorded comment and lists of alternative supplies and refills;
1) caregiver placing orders for suggested supplies and refills of medications; and
m) caregiver sending an electronic message to a medical personnel.

Clearly, the above method is facilitated by wireless communication, land line wire based communication and networked communication methods of well known types such as radio, telephone and Internet links, hook-ups, and other enablements.

As an example of the type of prerecorded information presented in a medical consultation style the following is presented. This video report would, in the present context, be viewed by a home caregiver, but might also be viewed by the patient him/herself or by both the caregiver and the patient. Referring to Figure 8, the video message for this patient record may be conducted as follows:

"Hello Bob, this is Nurse Brown from Remote Medical. Please wish your mother a happy birthday for me. I see that she is going to turn 80 later this week. I've reviewed your mother's compliance and vital signs reports. In the first half of the month your mother was very good about taking her medications and her blood glucose level was stable. You can see this on the graph just to the left. But then she went through a period in which she missed her medications and the blood glucose level rose too high and went into the red zone. She seems to be back on track right now. We know that your mother lives alone, so here's what I recommend that you do to help her. Review this chart with your mother and show her how the missed medications affected the blood glucose level. Then have her view the Patient Education Videos for Medication Compliance and Blood Glucose Levels on her WebTV. You can find the videos on the Patient Education page. I also looked at your mother's blood pressure readings. I don't think that the cuff is being put on the arm correctly. I also want you to sit down with her and review the on-line video that shows how this is done with her. Make sure that she can use the blood pressure monitor correctly on her own. Then we can track her blood pressure just like we've been tracking the blood glucose. I also looked at the images of the small ulcer on your mother's leg. It looks like its beginning to heal. Keep it clean and apply the ointment your doctor prescribed. It will probably take a few more weeks to fully heal. Let's see... your mother's file also indicates that she used her Emergency Alert Button once last week. We called her within 2 minutes and she told us that it was a false alarm. She had accidentally pressed the button while getting dressed. So we didn't notify you or your sister. This type of false alarm is very common and not a problem for us. Please be assured that we are monitoring her 24 hours a day. If she presses the Alert Button and anything is wrong, we will call you and the other people on the contact list immediately. Lastly, you need to purchase some additional supplies. It's been 30 days since your mother refilled her medications. Also, my computer is telling me that she only has enough Glucose Monitoring Strips for another 10 days. I recommend that you order 2 more boxes. Please press the "Place Order" button directly below me and authorize us to send her more medication and supplies. We can ship everything immediately. Also, if you would like, we can send your mother some flowers on her birthday with a card from you. You can optionally order this from the Refill and Supply page. If you have any questions, press the contact nurse button directly below me and send us an e-mail. Someone will get back to you within the hour. Bob, I know that we have covered a lot of ground in this report today. I encourage you to press the replay button directly below me and go through the list of items at least one more time. We know that you're very concerned about your mother's health. You're doing a good job of caring for her. Let us know if we can help. Good bye."

It is clear that the above method of communication is effective, surpassing audio only, or written communication methods in its ability to provide not only factual matter, but, quite importantly, in its ability to motivate to action. The message may be played over if necessary to further obtain information not understood, or simply missed on the previous playing.

While the invention has been described with reference to at least one preferred embodiment, it is to be clearly understood by those skilled in the art that the invention is not limited thereto. Rather, the scope of the invention is to be interpreted only in conjunction with the appended claims.

## Claims

1. A method for improving patient compliance with a medical program, the method comprising the steps:
a) providing a portable medical device means providing a medication storing means, a medication accessing means, a dispensed medication counting means, and a medical device wave energy communicating means;
b) sequencing a counter of the dispensed medication counting means each time the medication accessing means is manipulated for access to the medication storing means;
c) establishing a data set comprising each new numerical value of the counter and a corresponding time and date of the numerical value of the counter;
d) repetitively transmitting a wave energy signal corresponding to each newly established data set for a selected period of transmission time, the transmission time period chosen to assure a selected probability of the receipt of said wave energy signal by a base station.

2. The method of claim 1 further comprising the step of activating a human responsive signaling means in accordance with a medical schedule so as to provoke the scheduled and timely use of the portable medical device means.

3. The method of claim 1 further comprising the step of transmitting the medication schedule from the base station to the medical device means.

4. The method of claim 3 further comprising the step of transmitting the data set values of the medical device means to the base station.

5. The method of claim 4 further comprising the steps of providing a remote monitoring station; receiving the data set values from the base station at the remote monitoring station; computing a graphical correlation between the received data set values and a selected medical schedule, and transmitting the graphical correlation to the patient.

6. The method of claim 5 further comprising the step of composing a refill request message and of transmitting the refill request message to the patient.

7. The method of claim 5 further comprising the step of composing a graphical summary report showing a time sequence of medication dispensing over a selected time duration, and correlated thereto a time sequence of a corresponding medical data measurement over the selected time duration.

8. The method of claim 1 further comprising the steps of storing a list of all said data sets established over the selected period of transmission time; transmitting the list repetitively at a selected frequency of transmission.

9. The method of claim 1 further comprising the steps of pairing each of the data sets with a digital data integrity checking code and further providing an error-reducing protocol operating on the data sets as received for providing error reduction.

10. A method for improving patient compliance with a medical program, the method comprising the steps:
a) providing a portable medical device means providing a medical state sensing means, a medical state value recording means, and a medical device wave energy communicating means;
b) recording the medical state each time the medical state sensing means is manipulated for sensing the medical state;
c) establishing a data set comprising each new value of the medical state and a corresponding time and date of the numerical value of the counter;
d) repetitively transmitting a wave energy signal corresponding to each newly established data set for a selected period of transmission time, the transmission time period chosen to assure a selected probability of the receipt of said wave energy signal by a base station.

11. The method of claim 10 further comprising the step of activating a human responsive signaling means in accordance with a medical schedule so as to provoke the scheduled and timely use of the portable medical device means.

12. The method of claim 10 further comprising the step of transmitting the medical state sensing schedule from the base station to the medical device means.

13. The method of claim 12 further comprising the step of transmitting the data set values of the medical device means to the base station.

14. The method of claim 13 further comprising the steps of providing a remote monitoring station; receiving the data set values from the base station at the remote monitoring station; computing a graphical correlation between the received data set values and a selected medical schedule, and transmitting the graphical correlation to the patient.

15. The method of claim 10 further comprising the steps of storing a list of all said data sets established over the selected period of transmission time and then transmitting the list repetitively at a selected frequency of transmission.

16. The method of claim 10 further comprising the steps of pairing each of the data sets with a digital data integrity checking code and further providing an error-reducing protocol operating on the data sets as received for providing error reduction.

17. A method for creating video screens in a medical monitoring and control system comprising the sequenced steps:
a) logging a sequence of health event data into a data storage means at each of a plurality of time dependent data logging events;
b) transmitting each of a plurality of the sequence of health event data to a remote monitoring center in accordance with a preferred transmission schedule;
c) merging the health event data of the several sequences of health event data as a non-redundant set of such health event data;
d) producing a health status report in accordance with the non-redundant set of heath event data;
e) evaluating the health status report;
f) recording professional comments concerning the health status report as a video and audio presentation; and
g) adapting the recording and the health status report for electronic transmission on demand.

18. The method of claim 17 wherein the recorded comments comprise subject matter of at least one of: a familiarity commentary, a clinical review of graphical records, a health parameter trend information, a behavioral parameter trend information, and a needed products available for purchase information.

19. The method of claim 17 wherein the recorded comments comprise subject matter of at least one of: a positive behavioral reinforcement, an educational information, a purchasing information, at least one suggestion for improved patient behavior, and at least one of: clues, recommendations, acknowledgements and replies.

20. The method of claim 17 further comprising the step of producing a video screen comprising a first portion of the video screen depicting the health status report and a second portion of the video screen, adjacent to the first portion, depicting the professional comments recording.

21. The method of claim 17 wherein the comments recording comprises at least one of: behavior reinforcing comments, educational comments, and reassurance comments wherein the comments recording is directed for improving patient behavior.

22. The method of claim 17 wherein the comments recording comprises a summary of the health status report including medical significance and recommended changes in patient behavior and medications.

23. The method of claim 17 wherein the comments recording comprises at least one of: recommendations, suggestions, encouragements, demands, and clues for patient purchases.

24. The method of claim 17 further comprising the step of incorporating into the video screen, a plurality of action initiating button icons, and the further step of integrating the button icons with actions related to choices presented in the professional comments recording.

25. The method of claim 24 wherein at least one of the action initiating button icons is enabled for at least one of: a video presentation of medical products and supplies recommended for purchase and an initiating video screen for the purchase of the medical products and supplies recommended in the professional comments recording.

26. The method of claim 24 wherein at least one of the action initiating button icons is enabled for restarting the professional comments recording.

27. The method of claim 24 wherein at least one of the action initiating button icons is enabled for transmitting a message to a medical professional.

28. The method of claim 17 further comprising the step of incorporating into the video screen, a graphical representation of a medical device, and the further step of integrating the button icons with actions related to operation of the medical device.

29. The method of claim 17 further comprising the step of producing a video screen comprising a graphical listing of a plurality of commercial medical products related to the health status report and the professional comments and a graphical means for selecting any of said products on the screen.

30. The method of claim 29 wherein the video screen producing step further produces a graphical selection means for selecting a method of billing.

31. The method of claim 17 further comprising the additional step of notifying the patient that a video report is available for viewing.
